## Europäisches Patentamt
## European Patent Office
## Office européen des brevets

(11) Veröffentlichungsnummer: **0 266 590 B1**

(12) # EUROPÄISCHE PATENTSCHRIFT

(45) Veröffentlichungstag der Patentschrift: **24.04.91**

(51) Int. Cl.⁵: **A61M 5/14**

(21) Anmeldenummer: **87114998.5**

(22) Anmeldetag: **14.10.87**

(54) Infusionsvorrichtung.

(30) Priorität: **06.11.86 DE 3637771**

(43) Veröffentlichungstag der Anmeldung:
**11.05.88 Patentblatt 88/19**

(45) Bekanntmachung des Hinweises auf die
Patenterteilung:
**24.04.91 Patentblatt 91/17**

(84) Benannte Vertragsstaaten:
**AT BE CH DE ES FR GB GR IT LI LU NL SE**

(56) Entgegenhaltungen:
**EP-A- 0 111 842          DE-A- 2 526 865**
**DE-A- 3 439 661          FR-A- 2 417 300**
**US-A- 3 559 644          US-A- 3 965 895**
**US-A- 4 256 103**

(73) Patentinhaber: **B. Braun Melsungen AG**
**Carl-Braun Strasse**
**W-3508 Melsungen(DE)**

(72) Erfinder: **Mauerer, Erich**
**Druseltalstrasse 87**
**W-3500 Kassel(DE)**
Erfinder: **Mengel, Reiner**
**Lessingstrasse 5**
**W-3501 Niedenstein 3(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft eine Infusionsvorrichtung mit den Merkmalen des Oberbegriffs des Patentanspruchs 1. Eine derartige Infusions vorrichtung ist aurder DE-A-2 526 865 bekannt.

Zur Durchführung von Infusionen, d.h. zum Zuführen von Flüssigkeit zu einem Patienten, ist es bekannt, Schlauchpumpen zu verwenden, bei denen ein die Flüssigkeit enthaltender Schlauch fortlaufend abgequetscht wird, um die Flüssigkeit in dem Schlauch vorzutreiben. Die Genauigkeit der Förderrate hängt einerseits von der Genauigkeit der Drehzahl der Schlauchpumpe und andererseits von der Volumengenauigkeit des Schlauchs ab. Insbesondere Schläuche aus Weich-PVC haben eine geringe Volumengenauigkeit. Der Schlauch einer in einer Infusionsvorrichtung benutzten Schlauchpumpe ist ein Einmalartikel, der steril geliefert und im Anschluß an die Benutzung fortgeworfen wird. Bei solchen Schläuchen unterliegt die Volumengenauigkeit einerseits einer Exemplarstreuung und andererseits der Alterung und dem Verschleiß. Bei den bekannten Schlauchpumpen, bei denen der Schlauch ein kostengünstig herstellbarer Einmalartikel ist, beträgt die maximal erzielbare Genauigkeit der Förderrate etwa 5%. Eine solche Fördergenauigkeit ist für Infusionen von Medikamenten häufig zu gering.

Aus DE-A-25 26 865 ist ein Fluidströmungsregler für medizinische Anwendungen bekannt, bei dem aus einem Flüssigkeitsbehälter Flüssigkeit unter Schwerkraft in einen Strömungsdurchsatzregler gelangt, der ein Gehäuse mit zwei Kammern enthält. Die obere Kammer ist gegen die Umgebung hermetisch abgeschlossen und mit der unteren Kammer durch eine Öffnung von kleinem Querschnitt verbunden. Die untere Kammer ist belüftet und enthält an einem Auslaß ein Schwimmerventil. In der oberen Kammer stellt sich ein Flüssigkeitsstand ein, der bis zu dem Ende des in diese Kammer hineinragenden Zuführschlauchs reicht, während der Flüssigkeitsstand in der unteren Kammer vom Bedarf des Patienten bestimmt wird. Die in der oberen Kammer stehende Flüssigkeitssäule drückt Flüssigkeit durch die enge Öffnung hindurch in die untere Kammer hinein. An der unteren Kammer ist eine Elektrode zur Feststellung des Leerzustandes der unteren Kammer vorgesehen. Die bekannte Vorrichtung ist nicht imstande, einem Patienten Flüssigkeit mit konstanter Förderrate zuzuführen. Sie stellt lediglich eine begrenzte Flüssigkeitsmenge unter konstantem Druck für den Patienten bereit.

EP-A-0 111 842 beschreibt eine Dosiervorrichtung für die Schwerkraftzufuhr einer Flüssigkeit zu einem Patienten. Dabei wird der zum Patienten führende Schlauch von einem Exzenternocken zusammengedrückt. Die Zusammendrückung erfolgt dadurch, daß der Exzenternocken von einem Schrittmotor unter Steuerung durch einen Mikrocomputer verschwenkt wird. Ein Tropfensensor stellt die Zeit zwischen zwei aufeinanderfolgenden Flüssigkeitstropfen fest und liefert dieses Ergebnis an den Mikrocomputer, der daraufhin den Exzenternocken so verstellt, daß eine gewünschte Tropfrate entsteht. Damit kann zwar eine gewünschte Tropfrate erhalten werden, jedoch hängt die Förderrate unter anderem auch von der Tropfengröße ab. Da die Tropfengröße in weiten Bereichen variieren kann, ist die erzielbare Fördergenauigkeit entsprechend gering.

Der Erfindung liegt die Aufgabe zugrunde, eine Infusionsvorrichtung nach dem Oberbegriff des Patentanspruchs 1 derart weiterzubilden, daß die Infusion mit erhöhter Genauigkeit durchgeführt werden kann.

Die Lösung dieser Aufgabe erfolgt erfindungsgemäß mit den im kennzeichnenden Teil des Patentanspruchs 1 angegebenen Merkmalen.

Nach der Erfindung fördert die Pumpe die Flüssigkeit aus der Meßkammer heraus, deren Füllstand überwacht wird. Die Zeit, die erforderlich ist, um die Flüssigkeit von einem oberen Füllstand zu einem unteren Füllstand abzupumpen, wird gemessen und mit einer der Soll-Infusionsrate entsprechenden Soll-Entleerungszeit $t_S$ in Beziehung gesetzt. Wenn die tatsächliche Entleerungszeit von der Soll-Entleerungszeit abweicht, wird die Pumpengeschwindigkeit entsprechend korrigiert, so daß der nächstfolgende Entleerungsvorgang in einer Zeit erfolgt, die der Soll-Entleerungszeit angenähert ist, bzw. dieser entspricht. Unter "Entleerungszeit" soll die Zeit verstanden werden, die die Pumpe benötigt, um Flüssigkeit aus der Meßkammer von dem vorbestimmten oberen Füllstand bis zum vorbestimmten unteren Füllstand abzufördern. Bei dem unteren Füllstand ist noch Flüssigkeit in der Meßkammer enthalten, so daß diese in der Entleerungszeit nicht vollständig von Flüssigkeit entleert wird.

Im Anschluß an die Meßzeit, die gleich der Entleerungszeit ist, erfolgt in einer Füllzeit das Auffüllen der Meßkammer mit Flüssigkeit aus dem Flüssigkeitsbehälter bis zu dem vorgegebenen oberen Füllstand. Da die Förderrate von dem Flüssigkeitsbehälter zur Meßkammer wesentlich größer ist als die Infusionsrate, ist die Füllzeit viel kürzer als die Meßzeit.

Jeweils nach Ablauf der Meßzeit wird die Abweichung der Meßzeit von der Soll-Meßzeit festgestellt und in Abhängigkeit von dieser Abweichung wird die Pumpengeschwindigkeit korrigiert.

Die Genauigkeit der Infusionsrate hängt im wesentlichen von der Durchmessergenauigkeit der Meßkammer und der Genauigkeit des Füllstandsdetektors ab. Die Genauigkeit der Pumpe und die Volumengenauigkeit des Pumpenschlauchs werden eliminiert. Für den Meßbehälter und den Füllstandsdetektor lassen sich hohe Genauigkeiten erzielen, so daß die Genauigkeit der Infusionsrate bei ein bis zwei Prozent liegt. Dabei ist zu berücksichtigen, daß der Füllstandsdetektor fester Bestandteil des Gerätes sein kann und nicht als Wegwerfartikel ausgebildet sein muß. Für den Füllstandsdetektor kann daher ein größerer Aufwand betrieben werden, ohne daß die Kosten einer Infusion wesentlich erhöht werden. Die Meßkammer, die mit der Flüssigkeit in Berührung kommt, ist vorzugsweise Bestandteil einer Wegwerfeinheit. Sie kann als Kunststoffartikel relativ maßgenau hergestellt werden.

Obwohl die Flüssigkeit der Meßkammer in genau abgemessenen Volumenmengen intervallweise zugeführt wird, kann eine kontinuierliche Infusion zum Patienten erfolgen, wenn in der Füllzeit, die sich jeweils an eine Meßzeit anschließt, die Pumpe weiterläuft. Die Pumpensteuerung kann in der Füllzeit entweder mit der bisherigen Pumpengeschwindigkeit oder mit der am Ende der Meßzeit errechneten neuen Pumpengeschwindigkeit erfolgen.

Da die Förderrate einer Schlauchpumpe periodischen Schwankungen unterworfen ist, sollte jede Meßzeit mit einem vorbestimmten Phasenwinkel der Pumpenumdrehung beginnen. Die Pumpe sollte in der sich an die Meßzeit anschließenden Füllzeit bis zum Erreichen dieses Phasenwinkels weiterlaufen, d.h. die bei Erreichen des oberen Füllstandes begonnene Pumpenumdrehung sollte bis zum Erreichen des vorbestimmten Phasenwinkels vervollständigt werden, so daß die nächste Meßzeit wieder mit dem vorbestimmten Phasenwinkel beginnt.

Bei niedrigen Infusionsraten besteht auch die Möglichkeit, die Pumpe in der Füllzeit, in der die Meßkammer mit Flüssigkeit nachgefüllt wird, anzuhalten, falls die Infusion eine kurze Unterbrechung der Flüssigkeitszufuhr zuläßt.

Im folgenden werden unter Bezugnahme auf die Zeichnungen Ausführungsbeispiele der Erfindung näher erläutert.

Es zeigen:

Fig. 1

eine schematische Darstellung einer Ausführungsform der Infusionsvorrichtung,

Fig. 2

ein Zeitdiagramm der Förderrate der Pumpe,

Fig. 3

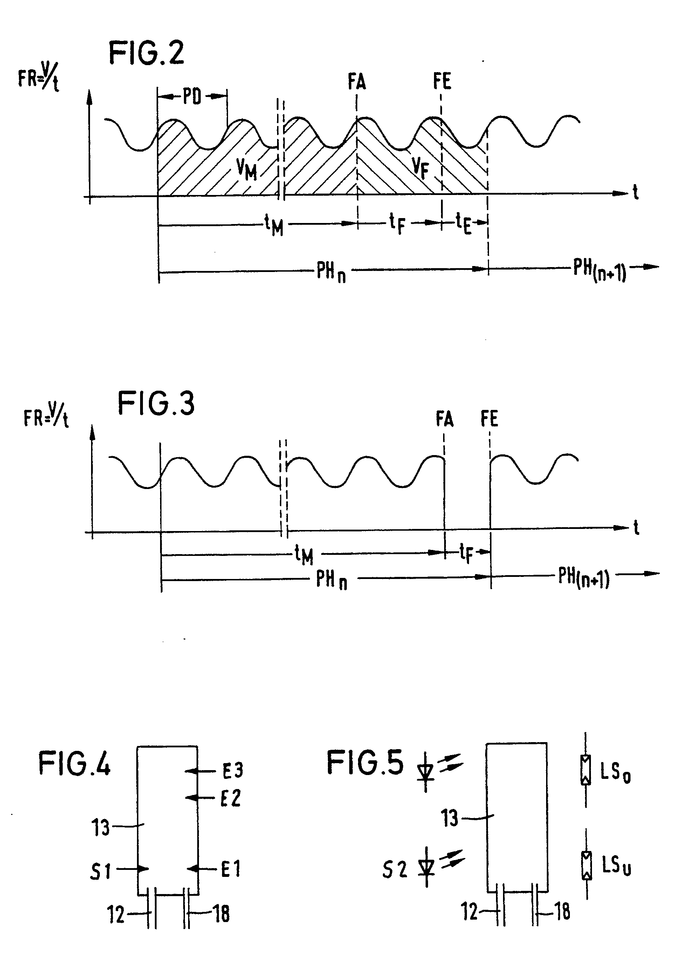

ein Zeitdiagramm ähnlich demjenigen der Fig. 2 bei einer anderen Betriebsart der Pumpe,

Fig. 4

eine modifizierte Ausführungsform der Meßkammer mit integriertem Füllstandsdetektor und

Fig. 5

eine weitere modifizierte Ausführungsform der Meßkammer mit externem Füllstandsdetektor.

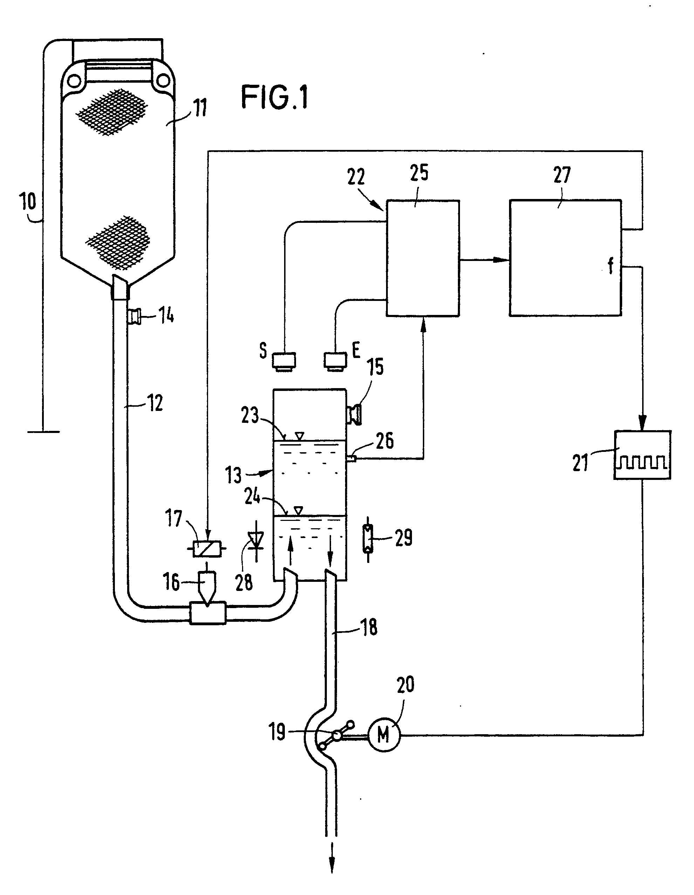

Gemäß Fig. 1 ist an einer Haltevorrichtung 10 der Flüssigkeitsbehälter 11 aufgehängt, der die Infusionslösung enthält. Das untere Ende des Flüssigkeitsbehälters 11 ist über einen Schlauch 12 mit der Meßkammer 13 verbunden, bei der es sich um einen aufrechtstehenden rotationssymmetrischen Hohlkörper handelt. Der Schlauch 12 führt in die Bodenwand der Meßkammer 13 hinein. Kurz unter dem Flüssigkeitsbehälter 11 ist an dem Schlauch 12 ein Belüftungsfilter 14 vorgesehen. Ein weiterer Belüftungsfilter 15 befindet sich in der Nähe des oberen Endes der Meßkammer 13. Der Flüssigkeitsbehälter 11 ist in einer solchen Höhe angeordnet, daß sein unteres Ende über dem Niveau des oberen Endes der Meßkammer 13 liegt, so daß Flüssigkeit aus dem Flüssigkeitsbehälter 11 in die Meßkammer 13 einfließen kann. Im Verlauf des Schlauchs 12 ist ein Quetschventil 16 vorgesehen, daß von einem Magneten 17 betätigt wird und den Schlauch 12 absperren kann.

Aus dem unteren Ende der Meßkammer 13 führt ein weiterer Schlauh 18 heraus zum Patienten. Im Verlauf des Schlauchs 18 ist die Schlauchpumpe 19 angeordnet, die als Rollenpumpe oder peristaltische Pumpe ausgebildet ist und mindestens zwei umlaufende Organe aufweist, welche einen Abschnitt des Schlauchs 18 fortlaufend abquetschen. Die Schlauchpumpe 19 wird von dem Motor 20 angetrieben.

Bei dem vorliegenden Ausführungsbeispiel ist der Motor 20 ein Schrittmotor, der von einem Impulsgenerator 21 mit Impulsen versorgt wird. Die Drehgeschwindigkeit der Schlauchpumpe 19 ist der von dem Impulsgenerator 21 gelieferten Impulsfrequenz proportional.

Der Füllstandsdetektor 22 erkennt den Füllstand der Meßkammer 13. Der Füllstandsdetektor 22 liefert ein erstes Signal, wenn ein bestimmter oberer Füllstand 23 erreicht ist und ein zweites Signal, wenn ein bestimmter unterer Füllstand 24 erreicht ist. Der Füllstandsdetektor 22 arbeitet bei dem vorliegenden Ausführungsbeispiel mit Ultraschallmessung. Eine Ultraschall-Meßeinrichtung 25 erregt einen Ultraschallsender S, der von oben her Ultraschallwellen in die Meßkammer 13 schickt. Diese Ultraschallwellen werden von dem jeweiligen Flüssigkeitsstand reflektiert und dem Empfänger E zugeführt. Aus der Laufzeit der

3

Ultraschallsignale bestimmt die Meßeinrichtung 25 die Höhe des jeweiligen Flüssigkeitspegels. Es gibt Ultraschall-Abstandsmesser mit einer Auflösung von ± 0,025 mm, so daß die beiden Flüssigkeitsstände 23 und 24 sehr genau erkannt werden können. Zur Eliminierung der Temperatureinflüsse (z.B. der Temperaturabhängigkeit der Schallgeschwindigkeit) ist an der Meßkammer 13 ein Temperatursensor 26 befestigt, der ein temperaturabhängiges Signal an die Meßeinrichtung 25 liefert, die daraufhin eine Temperaturkompensation ausführt.

Die Meßeinrichtung 25 liefert ein der Pegelhöhe in der Meßkammer entsprechendes Signal an das Steuergerät 27, welches die Frequenz f des Frequenzgenerators 21 und das Magnetventil 16, 17 steuert.

Gemäß Fig. 1 ist an der Meßkammer 13 eine fotoelektrische Sicherheitseinrichtung aus einer lichtemittierenden Diode 28 und einem Lichtempfänger 29 angeordnet. Die Sicherheitseinrichtung bildet eine Lichtschranke 28, 29, die erkennt, ob der Flüssigkeitspegel auf ein bestimmtes unteres Niveau, das unter dem Flüssigkeitsstand 24 liegt, abgesunken ist. Wenn dies der Fall ist, erfolgt eine Notabschaltung der Schlauchpumpe 19, um zu verhindern, daß Luft in den Körper des Patienten eingepumpt wird.

Beim Betrieb der in Fig. 1 dargestellten Vorrichtung wird zunächst das Quetschventil 16 geöffnet, so daß aus dem Flüssigkeitsbehälter 11 Flüssigkeit in die Meßkammer 13 fließt. Wenn der obere Füllstand 23 erreicht ist, schließt das Steuergerät 27 das Quetschventil 16. Die Schlauchpumpe 19 beginnt zu laufen und pumpt Flüssigkeit aus der Meßkammer 13 durch den Schlauch 18 zum Patienten.

Fig. 2 zeigt die Förderrate FR der Schlauchpumpe. Diese Förderrate ist definiert als das Fördervolumen V pro Zeit t. Man erkennt, daß die Förderrate sich periodisch verändert, wobei eine Periodendauer PD jeweils einer Umdrehung der Schlauchpumpe entspricht. Das Pumpvolumen $V_M$ während der Meßzeit $t_M$ entspricht dem Integral der Förderrate FR in der Meßzeit $t_M$. Dieses Integral ist in Fig. 2 durch die schräg nach rechts oben schraffierte Fläche dargestellt. Die Meßzeit $t_M$ ist beendet, wenn der untere Füllstand 24 erreicht ist. Dann beginnt unverzüglich die Füllzeit $t_F$ zum Zeitpunkt FA (Füllanfang). Die Füllzeit ist eine Größe, die sich aus der Länge des erforderlichen Füllvorgangs ergibt und die nicht vorherbestimmt ist. Sie endet zum Zeitpunkt FE (Füllende), wenn der obere Füllstand 23 erreicht ist. An die Füllzeit $t_F$ schließt sich dann noch eine Ergänzungszeit $t_E$ an, in der die Schlauchpumpe bis zum Ende der angefangenen Umdrehung weiterläuft, so daß dann die nächstfolgende Meßzeit mit definierter Phasenlage der Schlauchpumpe beginnen kann.

Die Meßzeit $t_M$, die die Schlauchpumpe 19 benötigt, um den Inhalt der Meßkammer 13 vom oberen Flüssigkeitsstand 23 auf den unteren Flüssigkeitsstand 24 abzusenken, wird im Steuergerät 27 gemessen. Ferner wird im Steuergerät 27 die für die nächste Phase (Summe aus den Zeiten $t_M$, $t_F$ und $t_E$) einzustellende Pumpengeschwindigkeit $v_{(n+1)}$ nach der Formel

$$v_{(n+1)} = \frac{T_{Mn}}{t_S} \cdot v_n \qquad (1)$$

berechnet. Hierin ist $v_n$ die Pumpengeschwindigkeit in der gerade ablaufenden n-ten Phase $PH_n$, $v_{(n+1)}$ die für die nächstfolgende Phase $PH_{(n+1)}$ einzustellende Pumpengeschwindigkeit, $T_{Mn}$ die Dauer der Meßzeit $T_M$ in der n-ten Phase und $t_S$ die Soll-Zeit, in der der Meßbehälter vom Füllstand 23 auf den Füllstand 24 entleert werden sollte.

Für die Soll-Zeit gilt

$$t_S = \frac{MV}{FR_S} \qquad (2)$$

Hierin ist MV das Meßvolumen der Meßkammer 13 zwischen den Füllständen 23 und 24 und $FR_S$ die Soll-Förderrate bzw. die Soll-Infusionsrate.

Bei dem beschriebenen Ausführungsbeispiel ist die Pumpengeschwindigkeit v proportional zur Frequenz f der dem Schrittmotor 20 zugeführten Impulse. Daher kann für die obige Gleichung gesetzt werden:

$$f_{(n+1)} = \frac{T_{Mn}}{t_S} \cdot f_n \qquad (3)$$

Hierin ist $f_n$ die Impulsfrequenz in der jeweils ablaufenden Phase $PH_n$ und $f_{(n + 1)}$ die für die nächstfolgende Phase $PH_{(n + 1)}$ einzustellende Frequenz.

Anstelle eines Schrittmotors 20 kann auch ein Gleichstrommotor oder ein anderer Motor mit steuerbarer Drehzahl benutzt werden. In einem solchen Fall ist dem Motor ein Positionsgeber nachgeschaltet, der die Drehstellung der Pumpenwelle mißt und dem Steuergerät 27 mitteilt. Die Pumpengeschwindigkeit wird dann von Phase zu Phase durch das Steuergerät 27 in gleicher Weise nachgeregelt wie bei dem in Fig. 1 dargestellten Ausführungsbeispiel.

Es sei angenommen, daß die eingestellte Förderrate $FR_S$ 600 ml/h beträgt. Das Volumen der Meßkammer 13 zwischen den Füllständen 23 und 24 betrage 15 ml. Die Förderzeit für das Abpumpen des Meßvolumens MV von 15 ml beträgt somit 90 s. Die Schlauchpumpe benötigt hierzu z.B. 34 Umdrehungen.

Die Pumpzeit $t_F$, die benötigt wird, um das Meßvolumen wieder aufzufüllen, beträgt in der Regel etwa 5 s. Dies bedeutet, daß die Schlauchpumpe 1,89 Umdrehungen ausführt, während das Meßvolumen nachgefüllt wird. In dieser Zeit werden 0,88 ml an Flüssigkeit gepumpt.

Der Schlauch 12 kann zwischen dem Flüssigkeitsbehälter 11 und dem Quetschventil 16 eine weitere Pumpe enthalten, um die Füllzeit der Meßkammer 13 zu verkürzen oder um eine erhöhte Anbringung des Flüssigkeitsbehälters 11 zu vermeiden.

Fig. 3 zeigt eine andere Betriebsart der Pumpe bei niedrigen Fördermengen. Hierbei ist die Meßzeit $t_M$ sehr lang und die Pumpe wird während der Füllzeit $t_F$ angehalten. Die Füllzeit ist abhängig vom Restvolumen des Flüssigkeitsbehälters 11 und wird daher gemessen. Zum Zeitpunkt FE beginnt die Pumpe mit demjenigen Phasenwinkel wieder zu laufen, bei dem sie angehalten hat. Eine Ergänzungszeit $t_E$ ist daher nicht erforderlich.

Die Soll-Zeit, in der das Meßvolumen aus der Meßkammer 13 abgepumpt werden soll, errechnet sich bei dieser Betriebsart zu

$$t_S = \frac{MV}{FR_S} - t_F. \qquad\qquad (2a)$$

Dadurch wird sichergestellt, daß die in der Meßzeit $t_M$ dem Patienten zugeführte Flüssigkeitsmenge für die Summe der Zeiten $t_M + t_F$ ausreicht, um die Soll-Förderrate $FR_S$ zu ergeben.

Fig. 4 zeigt eine andere Ausführungsform eines Füllstandsdetektors, bei dem mehrere Elektroden S1, E1, E2, E3 in der Wand der Meßkammer 13 enthalten sind. Jede dieser Elektroden kommt mit der Flüssigkeit in Berührung, wenn diese Flüssigkeit die Höhe der betreffenden Elektrode erreicht hat. Eine Sendeelektrode S1 legt ein Spannungspotential an die Flüssigkeit und wenn dieses Spannungspotential an einer der Empfangselektroden E1 bis E3 empfangen wird, ist dies das Kriterium dafür, daß die entsprechende Empfangselektrode mit Flüssigkeit benetzt ist. Die untere Empfangselektrode E1 befindet sich auf der Höhe des unteren Flüssigkeitsstandes 24 und die obere Empfangselektrode E3 befindet sich auf der Höhe des oberen Flüssigkeitsstandes 23. Unter der oberen Empfangselektrode E3 ist eine Hilfselektrode E2 angeordnet, um das Quetschventil 16 teilweise zu schließen, wenn die Flüssigkeit das Niveau der Hilfselektrode E2 erreicht hat. Auf diese Weise wird in der Endphase des Füllens der Meßkammer 13 ein langsamerer Flüssigkeitsanstieg erreicht, so daß ein Überschwappen über die Elektrode E3 verhindert wird.

Fig. 5 zeigt eine Ausführungsform des Füllstandsdetektors mit zwei Lichtschranken $LS_U$ und $LS_O$, deren Lichtstrahlen durch die durchsichtigen Wände der Meßkammer 13 hindurchgehen. Die Lichtstrahlen der Lichtschranken $LS_U$ und $LS_O$ sind jeweils scharf gebündelt. Auch hier ist eine Vorerkennung des oberen Füllstandes durch eine unterhalb der oberen Lichtschranke $LS_O$ angeordnete Hilfslichtschranke möglich.

Die Schläuche 12 und 18 sowie die Meßkammer 13 bilden zusammen mit dem Flüssigkeitsbehälter 11 eine Wegwerfeinheit, die steril verpackt geliefert wird und in das Gerät eingesetzt werden kann, um nach Beendigung der Infusion fortgeworfen zu werden.

**Ansprüche**

1. Infusionsvorrichtung mit
   - einem Flüssigkeitsbehälter (11),
   - einer Flüssigkeit aus dem Flüssigkeitsbehälter (11) empfangenden Meßkammer (13), deren Aus laß an einen Patienten anschließbar ist,

5

EP 0 266 590 B1

- und mindestens einem Füllstandsdetektor (22) zur Erkennung eines unteren und eines oberen Flüssigkeitsstandes der Meßkammer (13),
  **dadurch gekennzeichnet,**
- daß im Fließweg der Flüssigkeit hinter der Meßkammer (13) eine von einem Steuergerät (27) gesteuerte Schlauchpumpe (19) zum Zuführen der Flüssigkeit zum Patienten vorgesehen ist,
- das Steuergerät (27) bei Erreichen des unteren Füllstandes (24) während einer Füllzeit ($t_F$) die Flüssigkeitszufuhr von dem Flüssigkeitsbehälter (11) zur Meßkammer (13) durch Öffnen eines zwischen Flüssigkeitsbehälter (11) und Meßkammer (13) vorgesehenen Absperrorgans (16) freigibt und die Flüssigkeitszufuhr nach Erreichen des oberen Füllstandes (23) während einer Meßzeit ($t_M$) durch Schließen des Absperrorgans (16) unterbricht,
- und das Steuergerät (27) die Pumpengeschwindigkeit entsprechend der Abweichung der beim jeweils vorhergehenden Zyklus gemessenen Meßzeit ($t_M$) von einer der gewünschten Infusionsrate ($FR_s$) entsprechenden Soll-Zeit ($t_S$) erhöht oder verringert.

2. Infusionsvorrichtung nach Anspruch 1, dadurch gekennzeichnet, daß die Meßkammer (13) einschließlich der mit ihr verbundenen Schläuche (12, 18) eine Wegwerfeinheit bildet.

3. Infusionsvorrichtung nach Anspruch 1 oder 2, dadurch gekennzeichnet, daß die Schlauchpumpe (19) während der gesamten Füllzeit ($t_F$) mit der Geschwindigkeit ($v_n$) der vorhergehenden Meßzeit oder mit der neu berechneten Geschwindigkeit ($v_{(n + 1)}$) weiterläuft.

4. Infusionsvorrichtung nach einem der Ansprüche 1 bis 3, dadurch gekennzeichnet, daß jede Meßzeit $t_M$ mit einem vorbestimmten Phasenwinkel der Schlauchpumpe (19) beginnt und daß im Anschluß an die Meßzeit ($t_M$) und die Füllzeit ($t_F$) die Pumpe bis zum Erreichen dieses Phasenwinkels weiterläuft.

5. Infusionsvorrichtung nach Anspruch 1 oder 2, dadurch gekennzeichnet, daß die Schlauchpumpe (19) während der gesamten Füllzeit ($t_F$) gestoppt wird.

6. Infusionsvorrichtung nach einem der Ansprüche 1 bis 5, dadurch gekennzeichnet, daß das Absperrorgan (16) ein von dem Steuergerät (27) schaltbares Quetschventil ist.

## Claims

1. Infusion means comprising
   - a fluid container (11),
   - a measuring chamber (13) receiving fluid from said fluid container (11), the outlet of said measuring chamber being connectable to a patient, and
   - at least one filling level detector (22) for detecting a lower and an upper fluid level of said measuring chamber (13),
     characterized in
   - that a hose pump (19) for supplying fluid to a patient is arranged in the flow path of said fluid behind said measuring chamber (13), said hose pump being controlled by a control unit (27),
   - upon reaching the lower filling level (24) during a filling time ($t_F$), said control unit (27) releases the fluid supply from the liquid container (11) to the measuring chamber (13) by opening a closing member (16) arranged between said fluid container (11) and said measuring chamber (13), and, upon reaching the upper filling level (23), interrupts the fluid supply by closing said closing member (16) during a measuring time ($t_M$).
   - and that said control unit (27) increases or decreases the pump velocity according to the deviation in the measuring time ($t_M$), measured during the respective previous cycle, from the set time ($t_S$) corresponding to the desired infusion rate ($FR_s$).

2. The infusion means as defined in claim 1, characterized in that said measuring chamber (13) and the hoses (12, 18) connected thereto form a disposable unit.

3. The infusion means as defined in claim 1 or 2, characterized in that, during the total filling time ($t_F$), said hose pump (19) goes on operating at the speed ($v_n$) of the preceding measuring time or at the newly calculated speed ($v_{(n+1)}$)

6

**4.** The infusion means as defined in one of claims 1 to 3, characterized in that each measuring time ($t_M$) starts with a predetermined phase angle of said hose pump (19), and that, subsequent to the measuring time ($t_M$) and the filling time ($t_F$), said pump is further operative until said phase angle is reached.

**5.** The infusion means as defined in claim 1 or 2, characterized in that during the total filling time ($t_F$), said hose pump (19) is arrested.

**6.** The infusion means as defined in one of claims 1 to 5, characterized in that said closing member (16) is a squeezing valve switchable by said control unit (27).

**Revendications**

**1.** Dispositif d'infusion comprenant
   - un récipient à liquide (11),
   - une chambre de mesure (13), qui reçoit du liquide à partir du récipient (11) et dont la sortie peut être raccordée à un patient,
   - au moins un détecteur (22) du niveau de remplissage, servant à identifier un niveau inférieur et un niveau supérieur du liquide dans la chambre de mesure (13),
   caractérisé par le fait
   - que dans le trajet de circulation du liquide est prévue, en aval de la chambre de mesure (13), une pompe tubulaire (19) commandée par un appareil de commande (27) et servant à envoyer le liquide au patient,
   - lorsque le niveau inférieur de remplissage (24) est atteint, l'appareil de commande (27) libère, pendant une durée de remplissage ($T_F$), l'envoi du liquide du récipient à liquide (11) à la chambre de mesure (13) moyennant l'ouverture d'un dispositif de sectionnement (16) prévu entre le récipient à liquide (11) et la chambre de mesure (13) et, une fois que le niveau supérieur de remplissage (23) est atteint, interrompt l'envoi du liquide pendant une durée de mesure ($t_M$), par fermeture de l'élément, de sectionnement (16), et'
   - que l'appareil de commande (27) accroît ou réduit la vitesse de la pompe en fonction de l'écart entre la durée de mesure ($t_M$), mesurée lors du cycle précédent respectif, et la durée de consigne ($t_S$), qui correspond au débit désiré d'infusion ($FR_s$).

**2.** Dispositif d'infusion selon la revendication 1, caractérisé en ce que la chambre de mesure (13) forme, conjointement avec les tuyaux (12,30), qui sont raccordés à cette chambre, une unité jetable.

**3.** Dispositif d'infusion suivant la revendication 1 ou 2, caractérisé par le fait que la pompe tubulaire (19) continue à fonctionner pendant la totalité de la durée de remplissage ($t_F$) avec la vitesse ($v_n$) utilisée pendant la durée de mesure précédente ou avec la vitesse ($V_{(n+1)}$) nouvellement calculée.

**4.** Dispositif d'infusion selon l'une des revendications 1 à 3, caractérisé en ce que chaque durée de mesure ($t_M$) commence avec un angle de phase prédéterminé de la pompe tubulaire (19) et qu' à la suite de la durée de mesure ($t_M$) et de la durée de remplissage ($t_F$), la pompe continue à fonctionner jusqu'à ce que cet angle de phase soit atteint.

**5.** Dispositif d'infusion selon la revendication 1 ou 2, caractérisé en ces que la pompe tubulaire (19) est arrêtée pendant la totalité de la durée de remplissage ($t_F$).

**6.** Dispositif d'infusion selon l'une des revendications 1 à 5, caractérisé en ce que l'élément de sectionnement (16) est une soupape à pincement, pouvant être commutée par l'appareil de commande (27).

EP 0 266 590 B1

FIG.1

## FIG.2

$FR = \frac{v}{t}$

## FIG.3

$FR = \frac{v}{t}$

## FIG.4

## FIG.5